# EUROPÄISCHE PATENTSCHRIFT

(11) **EP 1 216 299 B1**
(45) Veröffentlichungstag und Bekanntmachung des Hinweises auf die Patenterteilung: **30.03.2005**
(21) Anmeldenummer: 00982946.6
(22) Anmeldetag: 27.09.2000
(51) Int. Cl.: C12N 15/00

(54) **GENTRANSFER IN HUMANE LYMPHOCYTEN MITTELS RETROVIRALER SCFV-ZELLTARGETING VEKTOREN**
GENE TRANSFER IN HUMAN LYMPHOCYTES USING RETROVIRAL SCFV CELL TARGETING VECTORS
TRANSFERT DE GENES DANS DES LYMPHOCYTES HUMAINS AU MOYEN DE VECTEURS RETROVIRAUX A CIBLAGE CELLULAIRE SCFC

(30) Priorität: 27.09.1999 DE 19946142
(43) Veröffentlichungstag der Anmeldung: 26.06.2002
(73) Patentinhaber: Bundesrepublik Deutschland, letztvertreten durch den Präsidenten des Paul-Ehrlich-Instituts Prof. Dr. Johannes Löwer, 63225 Langen (DE)
(72) Erfinder: CICHUTEK, Klaus, 63225 Langen (DE); ENGELSTÄDTER, Martin, 63322 Rödermark (DE)
(74) Vertreter: Grund, Martin, Dr.
(86) Internationale Anmeldenummer: PCT/DE2000/003444
(87) Internationale Veröffentlichungsnummer: WO 2001/025415

(56) Entgegenhaltungen:
- WO-A-96/36360
- WO-A-97/49726
- WO-A-98/51787
- DE-A- 19 752 854
- US-A- 5 844 094
- ENGELSTAEDTER MARTIN ET AL: "Targeting human T cells by retroviral vectors displaying antibody domains selected from a phage display library." HUMAN GENE THERAPY., Bd. 11, Nr. 2, 20. Januar 2000 (2000-01-20), Seiten 293-303, XP001004757 ISSN: 1043-0342

## Beschreibung

Die Erfindung betrifft den Gentransfer in humane Lymphocyten, insbesondere T-Lymphozyten mittels retroviraler scFv-Zelltargeting-Vektoren und die Verwendung dieser Vektoren zur Gentherapie, Impftherapie oder Diagnostik, insbesondere zur Therapie T-Zell-assoziierter Krankheiten.

Die Mehrheit der retroviralen Vektoren, die in der gentherapeutischen Forschung zur Zeit benutzt werden, stammen vom amphotropen Maus-Leukämie-Virus (MLV) ab. Der Wirtszellbereich des amphotropen MLV wird durch das Oberflächenhüllprotein (SU) bestimmt, das vom env-Gen codiert wird. Die Proteinprodukte des env-Gens bilden die äußere Hülle des retroviralen Vektors. Die SU-Proteine interagieren mit, d.h. sie binden an ein bestimmtes Protein (Rezeptor) auf der Oberfläche der Wirtszelle. Die env-Genprodukte des amphotropen MLV erlauben den Gentransfer in eine große Anzahl unterschiedlicher Säugerzellen. Ein selektiver Gentransfer in bestimmte Zell- oder Gewerbetypen des Menschen oder anderer Säuger ist mit amphotropen MLV-Vektoren aber nicht möglich, weil der Rezeptor für die MLV-Hüllproteine auf der Oberfläche der Säugerzellen, welches den Eintritt von amphotropen MLV-Vektoren und den Gentransfer vermittelt, auf fast allen diesen Zellen zu finden ist. Der Wirtszellbereich des amphotropen MLV ist daher nicht spezifisch.

Eine Wirtszellspezifität ist z.B. für den gentherapeutischen Einsatz jedoch von Vorteil, da bei einer Gentherapie außerhalb des Organismus (*ex vivo*) (Anderson et al. 1992; Yu et al., 1997) aufwendige Aufreinigungen von Zellen vermieden werden. Für den Therapie-, Diagnostikoder Impf-Einsatz *in vivo* ist erwünscht, daß die retroviralen Vektoren gezielt die gewünschten Wirtszellen ansteuern und anschließend das therapeutische Gen übertragen. Eine Einengung des Wirtszellbereichs des amphotropen MLV konnte durch Modifikation des Oberflächenhüllproteins erreicht werden. Eine Modifikation des Oberflächenhüllproteins wurde durch die Fusion mit einer Hormondomäne durchgeführt. Es fand eine Transduktion der Zellen statt, die den spezifischen Hormonrezeptor trugen (Kasahara et al., 1995). Ferner wurde das Oberflächenhüllprotein durch Fusion mit einem einkettigen Antikörperfragment (*single chain variable fragment,* nachfolgend auch "scFv" bezeichnet) modifiziert. Das Fragment repräsentierte die antigenbindende Domäne eines Antikörpers und ist ein Fusionsprotein, das aus den variablen Domänen Vh und V1 eines monoklonalen Antikörpers zusammengesetzt ist. Die beiden Domänen sind über ein Glycin- und Serin-Oligopeptid [-(sergly4)3-gly-)] verknüpft, das die korrekte Faltung des Fusionsproteins ermöglicht (Huston et al., 1991; Whitlow et al., 1991). Alle bisher durchgeführten Modifikationen des MLV-Oberflächenhüllproteins mit einem scFv zeigten, daß es zwar zu einer Bindung der Vektoren an die Wirtszielzelle kam, nicht jedoch zu einem Eintritt in die Zelle (Russel et al., 1993). Weiterhin ist bekannt, daß das Oberflächenhüllprotein des MLV generell keine umfangreichen Modifikationen erlaubt (Cosset et al., 1995). Modifikationen, bei denen ein Teil der Bindungsdomäne des MLV-SU-Proteins ersetzt wurde, führten oft zu einer inkorrekten Prozessierung und somit zu einem defekten Transport des SU-Proteins an die Zelloberfläche (Weiss et al., 1993; Morgan et al., 1993; Russel et al., 1993). Die Entwicklung zellspezifischer retroviraler Vektoren auf Basis des MLV mit veränderten Oberflächenhüllproteinen ist daher wenig erfolgversprechend.

Retrovirale Vektoren auf Basis des Milznekrosevirus SNV ("Spleen Necrosis Virus") sind für einen gezielten Gentransfer in z.B. humane Zellen geeigneter, da das Oberflächen-hüllprotein des SNV umfangreiche Modifikationen erlaubt und auch dann noch korrekt prozessiert wird (Martinez und Dornburg, 1995; Chu und Dornburg, 1994, 1995; Jiang et al., 1998). Zur Herstellung derartiger Vektoren benötigt man mindestens zwei Komponenten. Zum einen ist ein sog. Expressionskonstrukt herzustellen, das eine Verpackung in und den Transfer durch einen Retrovirus erlaubt. Das Expressionskonstrukt umfaßt ein kodierendes DNA-Fragment des gewünschten Genprodukts, z.B. ein Gen für die Gentherapie oder als Impfstoff. Das Expressionskonstrukt muß eine Nukleotidsequenz umfassen, die als Verpackungssignal psi (ψ) bezeichnet wird und die effiziente Verpackung der mRNA in retrovirale Partikel steuert. Ferner benötigt man eine Verpackungs- oder Helferzelle, welche die gag-, pol- und env-Genprodukte des SNV bereitstellt, ohne daß die gag-, pol- und env-Gene in ein Retrovirus verpackt werden können. Die in der Verpackungszelte befindlichen gag-, pol- und env-Gene müssen psi-negativ sein. Nach Überführung des Expressionskonstruktes durch Transfektion der entsprechenden Plasmid-DNA in die Verpackungszellen werden retrovirale Partikel in den Zellkulturüberstand abgegeben, die das Expressionskonstrukt enthalten und nur dieses, nicht jedoch die gag-, pol-, und env-Gene in die Zielzelle überführen können. Diese Vektoren sind vermehrungsunfähig und durchlaufen lediglich eine Replikationsrunde. Das allgemeine Verfahren zur Herstellung von vermehrungsunfähigen retroviralen Vektoren ist Stand der Technik (Russel et al., 1993; Cosset et al., 1995; Weiss et al., 1993; Morgan et al., 1993; Martinez und Dornburg, 1995; Chu und Dornburg, 1994, 1995; Jiang et al., 1998).

Auch der Tropismus (Wirtszellspezifität des Milznekrosevirus wird durch das Oberflächenhüllprotein (SU-Protein) bestimmt, das vom env-Gen des SNV codiert wird. Das Wildtyp-SNV-Oberflächenhüllprotein läßt keinen selektiven Gentransfer in bestimmte Zellen oder Gewebe des Menschen zu, da das spezifische Empfängerprotein (Rezeptor) nicht auf der Oberfläche von humanen Zellen vorhanden ist (Domburg, 1995). Deshalb wurde von Dornburg et al. ein Verfahren entwickelt, um das SU-Protein des SNV gegen die antigenerkennende Domänen von Antikörpern zu ersetzen. Diese [SNV-scFV-Env]-Vektoren mit vier bisher bekannten unterschiedlichen scFv waren in der Lage, das psi-positive Reportergen, die bakterielle β-Galaktosidase, in die ausgewählte humanen Zielzellen zu übertragen (Chu et al., 1994; Chu et al., 1995; Chu und Dornburg, 1997). Im einzelnen handelte es sich um zwei scFv, die gegen unbekannte Oberflächenantigene auf Brust- und Coloncarcinom Zellen exprimiert werden (Chu et al., 1995; Chu and Domburg, 1997; Jiang et al., 1998), um ein scFv, das gegen den humanen Transferrinrezeptor gerichtet ist und um ein scFv, das das CD34 Oberflächenantigen erkennt. Es wurde eine Verpackungszellinie (DSH-CXL) entwickelt, die sowohl die psi-negativen SNV-Gene gag, pol und env als auch das psi-positive Reportergen-Expressionskonstrukt (pCXL) enthält. Nach Transfektion der Verpackungszelle mit der Plasmid-DNA eines weiteren env-Expressionsgens (pTC53 [Expressionsvektor pTC53 und pTC53zeo Jiang et al., 1998] ), bei dem das gesamte Oberflächenhüllprotein gegen ein einkettiges Antikörperfragment (scFv) ersetzt wurde, wurden retrovirale Vektoren in den Zellüberstand abgegeben, die auf ihrer Oberfläche neben dem Wildtyp-Oberflächenhüllprotein auch das chimäre [scFv-Env]-Oberflächenprotein trugen. Mit Hilfe dieser Vektoren konnte das Reportergen in die für die scFv-spezifischen Zielzellen transferiert werden. Bei dem von Dornburg et al. beschriebenen Verfahren zur Herstellung zellspezifischer retroviraler Vektoren ist Fakt, daß nur bereits bekannte und klonierte scFv verwendet werden können.

DE 19752854 A1 beschreibt ein Verfahren zur Herstellung zelltypspezifischer, von SNVabgeleitete Targeting-Vektoren. Bisher sind 4 scFv-SNV-Targeting-Vektoren beschrieben worden. Diese sind gegen Tumormarker, den Transferrinrezeptor und gegen das CD34-Oberflächenantigen gerichtet (Chu & Dornburg, 1995, 1997, Jiang et al., 1997). Dabei wurden die scFv von monoklonalen Antikörpern (mAb) abgeleitet. Weiterhin sind bisher Pseudotypvektoren des Typs MLV (HIV) zur spezifischen Transduktion humaner CD4-positiver T-Zellen beschrieben worden (Schnierle & Stitz et al., 1997).

Allerdings sind bisher noch keine Vektoren beschrieben worden, die mit hoher Selektivität CD4-unabhängig humane T-Zellen transduzieren können.

Aufgabe der vorliegenden Erfindung war es daher T-Zell-spezifische Vektoren bereitzustellen, die CD4-unabhängig humane T-Zellen transduzieren können.

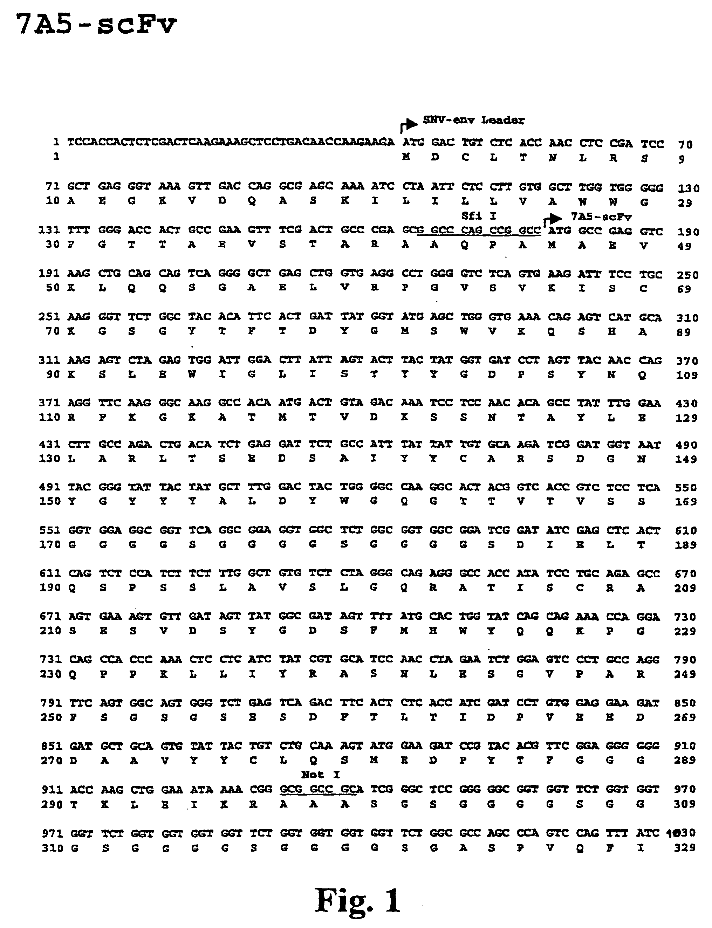

Die Aufgabe wird gelöst durch Zelltargeting-Vektoren, die eine DNA-Sequenz enthalten, die ein einkettiges Antikörperfragment (single chain variable fragment, scFv) codiert, wobei das einkettige Antikörperfragment eine Aminosäuresequenz gemäß der Figur 1 hat.

In einer bevorzugten Ausführungsform enthält der erfindungsgemäße Zelltargeting-Vektor weiterhin eine DNA-Sequenz, die einen SNV-env Leader gemäß der Figur 1 codiert. Die erfindungsgemäßen Zelltargeting-Vektoren sind T-Zell-spezifisch, d.h. die Vektoren induzieren selektiv CD4-unabhängig humane T-Zellen.

In einer weiter bevorzugten Ausführungsform ist der Zelltargeting-Vektor vom SNV (Milznekrosevirus) abgeleitet, besonders bevorzugt ist der von SNV abgeleitete Vektor pTC53.

In einer weiteren Ausführungsform der vorliegenden Erfindung enthalten die erfindungsgemäßen Zelltargeting-Vektoren ein therapeutisches Gen. Die Erfindung betrifft daher auch die Verwendung der erfindungsgemäßen Zelltargeting-Vektoren zur Gentherapie, Impftherapie oder Diagnostik.

Mit den erfindungsgemäßen scFv-Vektoren, stehen die ersten scFv- Zelltargeting-Vektoren zur Verfügung, die mit hoher Selektivität und unterschiedlich hoher Effizienz CD4-unabhängig humane T-Zellen transduzieren können.

Mittels der erfindungsgemäßen Vektoren ist es möglich folgende T-Zell-assoziierte Krankheiten zu therapieren:
(i) Schwere kombinierte Immunschwäche (SCID). Hierbei handelt es sich um einen Defekt des Adenosin-Desaminase-Gens (ada) oder des für die Thyrosinkinase JAK-3 kodierenden Gens (Macchi et al., 1995). Als therpeutisches Gen wird das intakte ada-Gen mittel der erfindungsgemäßen Vektoren in die T-Zellen transferriert.
(ii) Erworbene Immonschwäche (AIDS) wird durch die HIV-1 Infektion hervorgerufen. Therapeutische Gene sollen die Replikation oder Integration des Virus inhibieren. Als therapeutische Genprodukte für die intrazelluläre Immunisierung sind dabei Ribozyme, Köder-RNA, transdominant negative Mutanten von HIV-Proteinen oder Antikörperfragmente geeignet (Chang et al., 1994, Ramenzani et al., 1997, Smith et al., 1996, Leavitt et al., 1996, Duan et al., 1995, Levy-Mintz et al., 1996). Diese therapeutischen Gene werden bei der erfindungsgemäßen Verwendung der neuen Zelltargeting-Vektoren in die T-Zellen HIV-1 infizierter Patienten transferriert.
   Es konnte gezeigt werden, daß mittels der erfindungsgemäßen Vektoren (z.B Vektoren, enthaltend das in Fig. 1 gezeigte scFv 7A5; nachfolgend als 7A5-Vektoren bezeichnet) humane Makrophagen mit einer Effizienz von 95% transduziert werden. Somit ist mittels dieser 7A5-Vektoren der Transfer therapeutischer Gene auch in HIV-1 infizierte Makrophagen möglich.
(iii) T-Zell assoziierte Lymphome.

Die erfindungsgemäßen (scFv-SNV-Env)-Targetingvektoren, enthaltend eine DNA-Sequenz codierend ein einkettiges Antikörperfragment (single chain variable fragment, scFv), wobei das einkettige Antikörperfragment eine Aminosäuresequenz (oder ein Fragment) gemäß der Figur 1 hat, ermöglichen selektiv eine Transduktion humaner T-Zellinien und zum Teil aus dem Blut isolierter primärer Lymphocyten.

Überraschenderweise zeigen die erfindungsgemäßen Vektoren eine um das Vielfache erhöhte Selektivität für humane T-Zellen im Vergleich zu anderen humanen Zellen. Die 7-A5-Vektoren, d.h. die Vektoren, die das einkettige Antikörperfragment gemäß Fig. 1 oder einen Teil hiervon kodieren, zeigten eine bis um den Faktor 1000 erhöhte Selektivität für humane T-Zellen im Vergleich zu anderen humanen Zellen besitzt (s. Tabelle 2) und eine 4-5 fach erhöhte Selektivität für T-Zellen im Vergleich zu B-Zellen.

In Tabelle 1 sind 5 scFv (im einzelnen: 7A5, K6, 7B2, 7E4, 6C3) und ihre Vektortiter auf humanen T-Zellen (C8166), D17 Zellen (Hunde Osteosarkom-Zellinie, permissiv für SNV) und HeLa Zellen (humane Zervixkarzinom-Zellinie) dargestellt.

In Tabelle 2 sind die Vektortiter von 7A5-Vektoren dargestellt. Aus diesen Daten ist die Effizienz und Spezifität für humane T-Zellen erkennbar. Mittels dieser 7A5-Vektoren konnten auch mittels gentechnisch veränderter SNV-Vektoren ruhende T-Zellen und sogar humane Makrophagen sehr effizient transduziert werden.

Die folgenden Beispiele erläutern die Erfindung und sind nicht als einschränkend aufzufassen:

### Beispiel 1:

### Bestimmung der Vektortiter der fünf selektionierten scFv auf D17-. C8166- und HeLa-Zellen.

Hierzu wurden die Zellkulturüberstände in drei Verdünnungsstufen (1000 µl, 100 µl und 10µl) in einem Gesamtvolumen von 1000µl unter Zugabe von 30 µg/ml Polybren auf den Zellen (2 x 105 D17 und HeLa, 5 x 105 C8166) titriert. Nach 1,5-2 h Inkubationszeit wurde der vektorhaltige Überstand durch frisches Medium ersetzt.

Nach 48 h wurde zur Detektion der transduzierten Zellen eine X-gal Anfärbung durchgeführt (Mikawa et al., 1992) und die blauen Zellen gezählt. Tab. 1 zeigt die Vektortiter der fünf selektionierten scFv auf D17-, C8166- und HeLa-Zellen .

Die Titration auf D17 (Hundeosteosarkomzellinie, Watanabe et al., 19) dient als positiv-Kontrolle für die Vektorproduktion. Der Titer von >10⁶ i.E./ml zeigt, dass alle 5 scFv-Verpackungszellklone mit ungefähr gleicher Effizienz Vektorpartikel in den Zellkulturüberstand abgeben.

Die Titer auf C8166 Zellen schwanken zwischen 10³ und 10⁶ i.E./ml je nach scFv während die Transduktion auf HeLa Zellen keinen nennenswerten Titer ergab. Diese Tatsache deutet auf eine hohe Selektivität für humane T-Zellen aller fünf scFv-Vektoren hin. Die 7A5 Vektoren transduzieren am effizientesten humane T-Zellen (Tabelle 1).

**Tab. 1:**

| Vektortiter der fünf scFv-Vektoren | | | |
|---|---|---|---|
| Titer (i.E./ml) | | | |
| ScFv | D17 | C8166 | HeLa |
| 7A5 | >10⁶ | 1x10⁶ | <10² |
| K6 | >10⁶ | 2,5 x 10⁵ | <10¹ |
| 7B2 | >10⁶ | 2 x 10⁴ | <10¹ |
| 7E4 | >10⁶ | 2 x 10³ | <10¹ |
| 6C3 | >10⁶ | 2 x 10³ | <10¹ |

### Beispiel 2:

### Weitere Charakterisierung der Vektoren

Zur genaueren Charakterisierung werden weitere Transduktionsexperimente mit den Vektoren durchgeführt. In Tabelle 2 sind die Ergebnisse der 7A5-Vektoren dargestellt.

**Tab. 2:**

| Transduktion verschiedener Zelltypen mittels 7A5- und Wildtyp-Vektoren | | | | | | | | | | |
|---|---|---|---|---|---|---|---|---|---|---|
| Titer (i.E/ml) | | | | | | | | | | |
| | D17 | HeLa | TE671 | HT1080 | 293T | C8166 | Molt4/8 | Jurkat | A301 | huPBMC |
| wt | >10⁶ | <10¹ | <10¹ | <10¹ | <10¹ | <10¹ | <10¹ | <10¹ | <10¹ | <10¹ |
| 7A5 | >10⁶ | <10² | <10¹ | <10¹ | <10² | 1x10⁵ | 1x10⁶ | 3x10⁵ | 1x10⁵ | 7,5x10⁴ |

Die Transduktionen wurden wie oben beschrieben durchgeführt. Als Kontrolle wurden alle Zellen mit Wildtyp-Vektoren (wt) transduziert. Hierbei handelt es sich um Vektorpartikel, die nur das Wildtyp-SNV-Env-Protein und kein scFv besitzen. Diese werden von der Ausgangs-Verpackungszellinie DSH-cx1 (Chu & Dornburg, 1995, Jiang et al., 1998) in den Kulturüberstand abgegeben. Erwartungsgemäß konnten diese Vektoren humane Zellen nicht transduzieren. Nur die für sie permissiven D17 Zellen konnten mit hoher effizient transduziert werden.

Die Titration mit 7A5-Vektoren zeigte eine effiziente Transduktion von mehreren humanen T-Zellinien (C8166, Molt4-8, Jurkat, A301), während andere humane Zelltypen (HeLa: Cervixcarcinom, TE671: Rhabdomyosarkom, HT1080: Fibrosarkom, 293T: Nierenmark) nicht transduziert wurden. Diese Ergebnisse zeigen, daß die 7A5-Vektoren eine hohe Selektivität für T-Zellen besitzt.

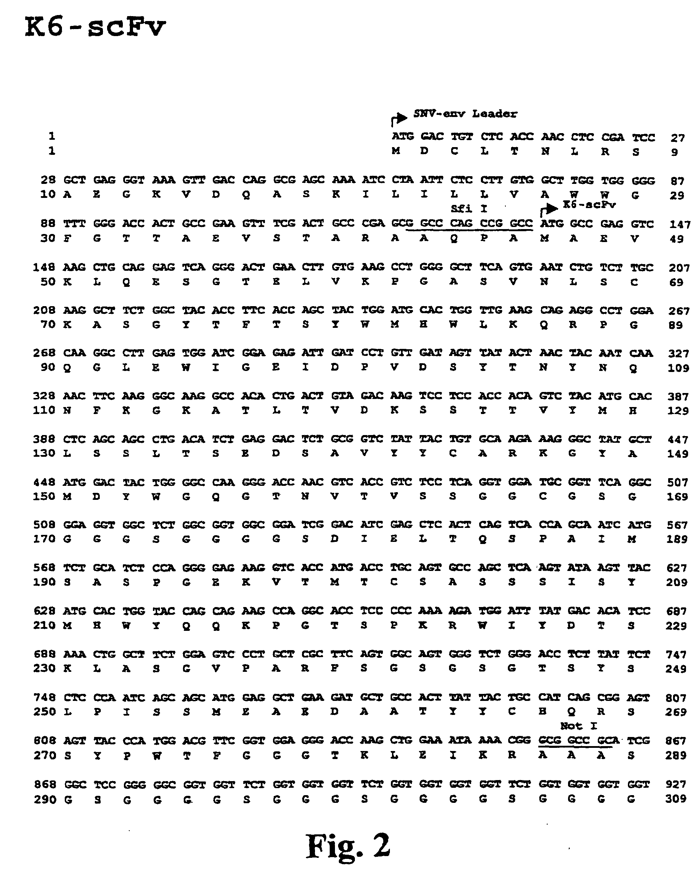

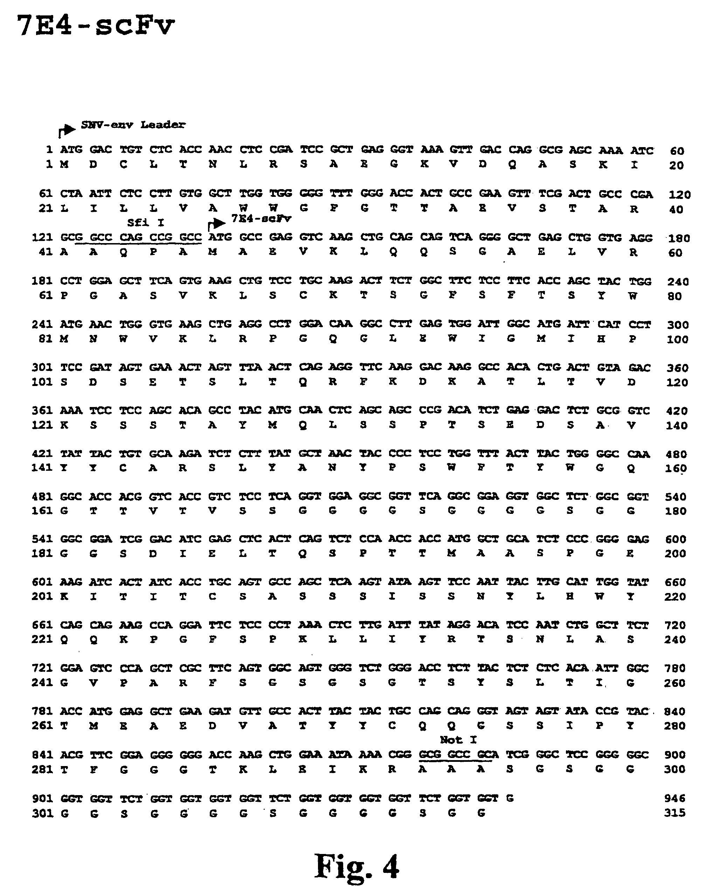

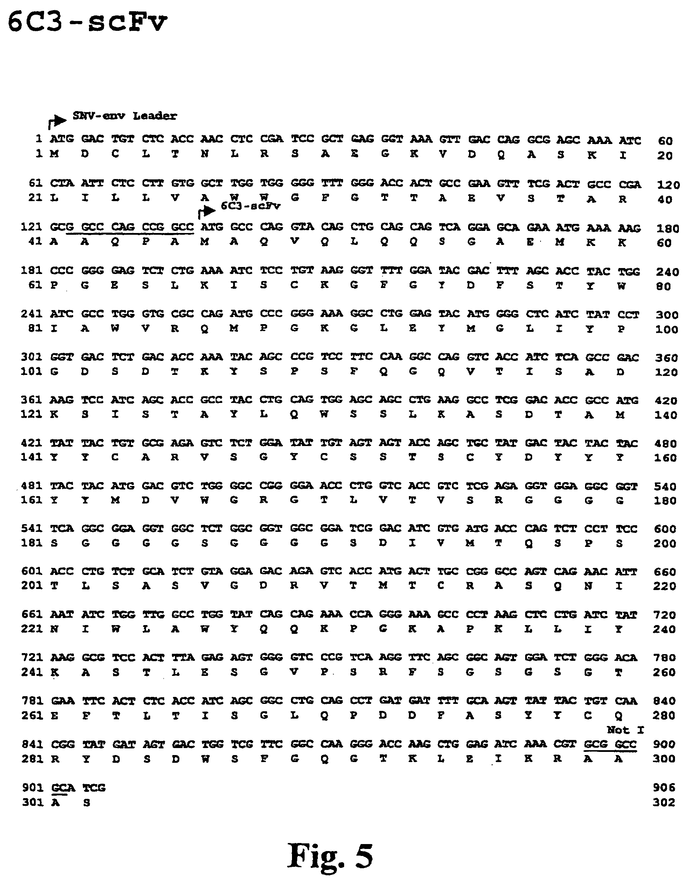

Eine erhöhte Selektivität für T-Zellen wurde auch für Zelltargeting-Vektoren gefunden, die eine DNA-Sequenz enthalten, die für ein einkettiges Antikörperfragment gemäß Figur 2, 3, 4 oder 5 codiert.

### Beispiel 3:

### Transduktion von primären T-Zellen

Zur Transduktion von primären T-Zellen wurden aus Blut primäre humane PBMC ("periphäre mononuleäre Zellen", die Isolierung von PBMC aus Blut mittels Sucrose-Dichtegradient-Zentrifugation erfolgt nach Standardtechniken) isoliert.

Nach dreitägiger Stimulierung mittels PHA (Phytohämagglutinin) und IL-2 bestand die Zellpopulation aus 98% T-Lymphozyten (ermittelt mittels FACS-Analyse mit einem Antikörper gegen den T-Zellmarker CD3 (Stand der Technik)).

Die Transduktion dieser Zellen mittels 7A5-Vektoren ergab eine Effizienz von 20% Vektorpositiver Zellen (oder ca. 1x10⁵ i.E./ml). Zum Vergleich wurden Transduktionsexperimente mit humanen B-Zellen durchgeführt. Diese konnten etwa fünf-fach schwächer ( ca. 4%) transduziert werden als T-Zellen.

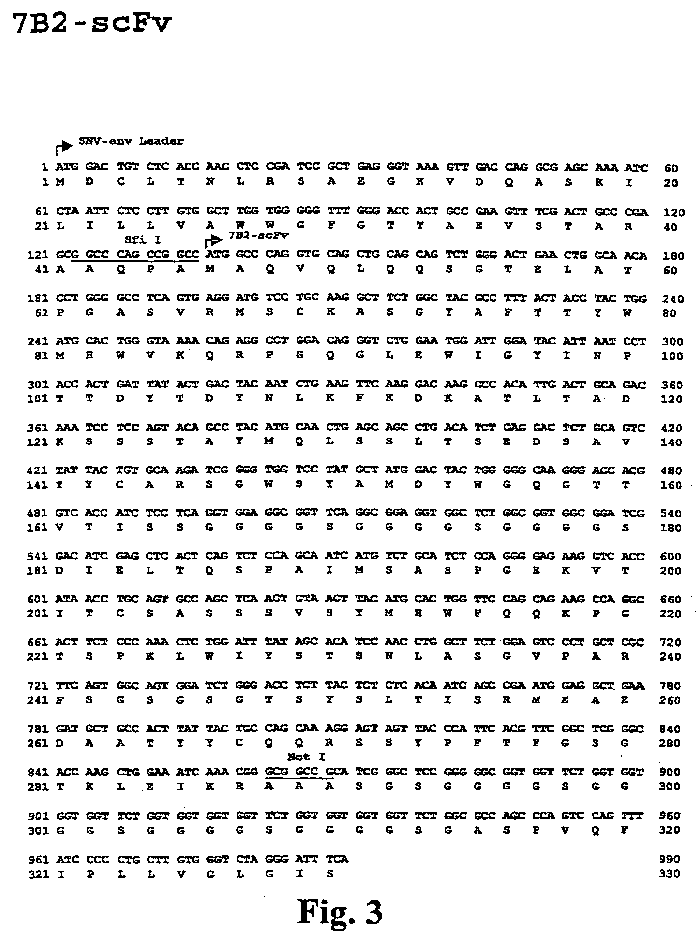

Stimulierter humane PBMC konnten ferner auch mit den K6- und 7B2-Vektoren (d.h Vektoren, die das einkettige Antikörperfragment gemäß Fig. 2 oder 3 oder einen Teil hiervon kodieren,) transduziert werden. Dies jedoch mit einer etwa 10 fach niedrigeren Effizienz als mit den 7A5-Vektoren.

### Literaturliste

ANDERSON, (1992) Human Gene Therapy. Science 256: 808-813
Chang H.K., Gendelman R., Lisziewicz J., Gallo R.C., Ensoli B. (1994). Block of HIV-1 infection by a combination of antisense tat RNA and TAR decoys: a strategy for control of HIV-1. Gene Therapy 1: 208-216
CHU, T.-H. and DORNBURG, R. (1995). Retroviral vector particles displaying the antigenbinding site ofan antibody enable cell-type-specific gene transfer. J. Virol. 69, 2659-2663
CHU, T.-H. and DORNBURG, R. (1997). Toward highly efficient cell-type-specific genetransfer with retroviral vectors displaying single-chain antibodies. J. Virol. 71, 720-725
CHU, T.-H.-T., MARTINEZ, I., SHEAY, W., DORNBURG R (1994). Cell targeting with retroviral vector particles containing antibody-Envelope fusion proteins. Gene Therapie 1: 292-299
COSSET, F., MORLING, F., TAKEUCHI, Y., WEISS, R, COLLINS, M., RUSELL, S. (1995). Retroviral Retargeting by Envelopes Expressing an N-terminal Binding Domain. J. Virol 69, No. 10: 6314-632
Duan L., Zhu M., Bagasra O., Pomerantz R.J. (1995). Intracellular immunization against HIV-1 infection of human T lymphocytes: utility of anti-Rev single-chain variable fragments. Hum. Gene Ther. 6: 1561-1573
DORNBURG, R (1995). Reticuloendoteliosis viruses and derived vectors. Gene Therapie 2: 1-10
ENGELSTÄDTER, M., BOBKOVA, M., BAIER, M, STITZ, J., HOLTKAMP, N., CHU, T.-H.-T., KURTH, R., DORNBURG, R., BUCHHOLZ, C. J., AND CICHUTEK, K. Targeting human T-cells by retroviral vectors displaying antibody domains selected from a phage display library. (submitted tu Human Gene Therapy)
HUSTON, J. S., MUDGETT-HUNTER, M., TAI, M. S., MCCARTHNEY, J., WARREN, F., HABER, E., (1991). Protein engineering of single-Chain Fv proteins and fusion proteins. Methods Enzymol. 203:46-88
JIANG A., CHU, T.-H., NOCKEN, F., CICHUTEK, K., and DORNBURG, R (1998). Celltype specific gene transfer into human cells with retroviral vectors that display single-chain antibodies. J. Virol. 72,10148-10156
KASAHARA, N., DOZY, A. M., YUET WAI KAN (1994). Tissue-Specific Targeting of Retroviral Vectors Through Ligand-Receptor Interactions. Science 266: 1373-1375
Leavitt M.C., Wong-Staal F., Looney D.J. (1996). Ex vivo transduction and expansion of CD4+ lymphocytes from HIV+ donors: a prelude to a ribozyme gene therapy trial. Gene Ther. 7: 599-606
Levy-Mintz P., Duan L., Zhang H., Hu B., Dornadula G., Zhu M., Kulkoski J., Bizub-Bender D., Skalka A.M., Pomeranz R.J. (1996). Intracellular expression of single-chain variable fragments to inhibit early stages ofthe viral life cycle by targeting human immunodeficiency virus type integrase. J. Virol. 70: 8821-8832
Macchi P., Villa A., Giliani S., Sacco M.C., Frattini A., Port F., Ugazio A.G., Jonston J.A., Candotti F., O Shea J.J., Vezzoni P., Notarangelo L.D. (1995). Mutations ofthe JAK-3 gene in patients with autosomal sever conmined immune deficiency (SCID). Nature 377: 65-68
MARTINEZ, I. and DORNBURG, R. (1995). Improved retroviral packaging cell lines derived from spleen necrosis virus. Virology 208, 234-241
MARTINEZ, I., DORNBURG, R (1995). Mapping of Receptor Binding Domains in the Envelope Protein of Spleen Necrosis Virus. J. Virol. 69, No. 7
MIKAWA, T., FISCHMANN. D. A., DOUGHERTY, J. P., and BROWN, A. M. C. (1992). In vivo analysis of a new lacZ retrovirus vector suitable for lineage marking in avian and other species. Exp. Cell Res. 195, 516-523
MORGAN, R. A., Nussbaum, O., Muenchau, D.D., Shu, L., Coutre, L., Andeson, W.F. (1993). Analysis of the functional and the host range-determining regions of the murine ecotropic and amphotropic retrovirus envelope proteins. J. Virol. 67: 4712-4721
PARVEEN, Z., KRUPETZKI, A., POMERANTZ, R. J., ENGELSTÄDTER, M., CICHUTEK, K., AND DORNBURG, R. Genetically engineered c-type retroviral vectors, derived from spleen necrosis virus, SNV, capable of infecting quiscent cells. (submitted to Nature Biotechnology)
Ramenzani A., Ding S.F., Joshi S. (1997). Inhibition of HTV-1 replication by retroviral vectors expressing monomeric and multimeric hammerhead ribozymes. Gene Ther. 4: 861-867
RUSSEL, S. J., HAWKINS, RE., WINTER, G. (1993). Retroviral vectors displaying functional antibody fragments. Nucleic Acid Res.21: 1081-1985
SCHNIERLE, B. S., STITZ, J., BOSCH, V., NOCKEN, F., MERGET-MILLITZER, H., ENGELSTADTER, M., KURTH, R., GRONER, B., CICHUTEK, K. (1997). Pseudotyping of murine leukemia virus with the envelope glycoproteins of HIV generates a retroviral vector with specificity of infection for CD4-expressing cells. Proc Natl Acad Sci USA 94(16):8640-8645.
Smith C., Lee S.W., Wong E., Gallardo H., Page K., Gaspar O., Lebowski J., Gilboa E. (1996). Transient protection of human T-cells from human immunodeficiency virus type 1 infection by transduction with adeno-associated viral vectors which express RNA decoys. Antiviral Res. 32: 99-115
WATANABE, S. AND TEMIN, H. M. (1983). Construction of a helper cell line for avian reticuloendotheliosis virus cloning vecotrs. Moll. Cell Biol. 3: 2241-2249
WEISS, R (1993). Cellular receptors and viral glycoproteins involved in retroviral entry. In J.A.Levy (ed.). The Retroviridae 2: 1-108
WHITLOW, M. AND FILPULA, D., (1991). Single-Chain Fv proteins snd their fusion proteins. Methods: Acompanion to Methods Enzymol. 2:97-105
YU, J. S., BURWICK, J. A., DRANOFF, G., BREAKEFIELD, X., (1997). Gene Therapy for metastatic Brain Tumors by Vaccination with Granulocyte-Macrophage-Colony-Stimulation Factor-Transduced Tumor Cells. H. Gene Therapy 8:1065-1072

## Patentansprüche

1. Zelltargeting-Vektor enthaltend eine DNA-Sequenz codierend ein einkettiges Antikörperfragment (single chain variable fragment, scFv) **dadurch gekennzeichnet, daß** das einkettige Antikörperfragment eine Aminosäuresequenz gemäß Figur 1 hat.

2. Zelltargeting-Vektor gemäß Anspruch 1, **dadurch gekennzeichnet, daß** der Vektor weiterhin eine DNA-Sequenz enthält, die einen SNV-env Leader gemäß Figur 1 codiert.

3. Zelltargeting-Vektor gemäß Anspruch 1 oder 2, **dadurch gekennzeichnet, daß** der Vektor T-Zell-spezifisch ist.

4. Zelltargeting-Vektor gemäß einen der Ansprüche 1 bis 3, **dadurch gekennzeichnet, daß** der Vektor vom SNV (Milznekrosevirus) abgeleitet ist

5. Zelltargeting-Vektor gemäß Anspruch 4, **dadurch gekennzeichnet, daß** der von SNV abgeleitete Vektor pTC53 ist.

6. Zelltargeting-Vektor nach einem der Ansprüche 1 bis 5 enthaltend ein therapeutisches Gen.

7. Arzneimittel, enthaltend Zelltargeting-Vektoren gemäß einem der Ansprüche 1 bis 6.

8. Verwendung der Zelltargeting-Vektoren gemäß einem der Ansprüche 1 bis 6 zur Herstellung eines pharmazeutischen Zusammasetzung zur Gentherapie, Impftherapie oder Diagnostik.

9. Verwendung der Zelltargeting-Vektoren gemäß einem der Ansprüche 1 bis 6 zur Herstellung eines pharmazeutischen Zusammasetzung zur Therapie T-Zell-assoziierter Krankheiten.

10. Verwendung gemäß Anspruch 8, wobei die T-Zell-assoziierte Krankheit erworbene Immunschwäche (AIDS) oder schwere kombinierte Immunschwäche (SCID) ist.

## Claims

1. A cell targeting vector, containing a DNA sequence encoding a single chain antibody fragment (single chain variable fragment, scFv), **characterized in that** the single chain antibody fragment has an amino acid sequence according to Figure 1.

2. The cell targeting vector according to claim 1, **characterized in that** the vector further contains a DNA sequence encoding a SNV-env leader according to Figure 1.

3. The cell targeting vector according to claim 1 or 2, **characterized in that** the vector is T cell specific.

4. The cell targeting vector according to any one of claims 1 to 3, **characterized in that** the vector is derived from SNV (Spleen Necrosis Virus).

5. The cell targeting vector according to claim 4, **characterized in that** the vector derived from SNV is pTC53.

6. The cell targeting vector according to any one of claims 1 to 5, containing a therapeutic gene.

7. A pharmaceutical composition containing cell targeting vectors according to any one of claims 1 to 6.

8. The use of the cell targeting vectors according to any one of claims 1 to 6 for the preparation of a pharmaceutical composition for gene therapy, vaccination therapy or diagnostics.

9. The use of the cell targeting vectors according to any one of claims 1 to 6 for the preparation of a pharmaceutical composition for the therapy of T-cell associated diseases.

10. The use according to claim 8, wherein the T-cell associated disease is Acquired Immunodeficiency Syndrome (AIDS) or Severe Combined Immune Deficiency (SCID).

## Revendications

1. Vecteur de ciblage de cellules, contenant une séquence d'ADN codant pour un fragment d'anticorps à chaîne unique (single chain variable fragment, scFv), **caractérisé en ce que** le fragment d'anticorps à chaîne unique a une séquence d'acides aminés selon la Figure 1.

2. Vecteur de ciblage de cellules selon la revendication 1, **caractérisé en ce que** le vecteur contient en outre une séquence d'ADN qui code pour un leader SNV-env selon la Figure 1.

3. Vecteur de ciblage de cellules selon la revendication 1 ou 2, **caractérisé en ce que** le vecteur est spécifique des cellules T.

4. Vecteur de ciblage de cellules selon l'une des revendications 1 à 3, **caractérisé en ce que** le vecteur est dérivé de SNV (virus de la nécrose de la rate).

5. Vecteur de ciblage de cellules selon la revendication 4, **caractérisé en ce que** le vecteur dérivé du SNV est pTC53.

6. Vecteur de ciblage de cellules selon l'une des revendications 1 à 5, contenant un gène thérapeutique.

7. Médicament contenant des vecteurs de ciblage des cellules selon l'une des revendication 1 à 6.

8. Utilisation des vecteurs de ciblage des cellules selon l'une des revendications 1 à 6 pour la préparation d'une composition pharmaceutique pour la thérapie génique, la thérapie par inoculation ou le diagnostic.

9. Utilisation de vecteurs de ciblage de cellules selon l'une des revendications 1 à 6, pour la préparation d'une composition pharmaceutique pour la thérapie d'affections associées aux cellules T.

10. Utilisation selon la revendication 8, dans laquelle l'affection associée aux cellules T est le syndrome d'immunodéficience acquise (SIDA) ou le syndrome d'immunodéficience combinée lourde (SIDC).


### SEQUENZPROTOKOLL

<110> Bundesrepublik Deutschland, letztvertreten durch den Präsidenten des Paul-Ehrlich-Instituts Prof. Dr. R. Kurth, 63225 Langen
<120> Gentransfer in humane Lymphocyten mittels retroviraler scFv-Zelltargeting Vektoren
<130> 158-6 PCT
<140> PCT/DE
   <141> 2000-09-27
<150> DE 199 46 142.2
   <151> 1999-09-27
<160> 10
<170> PatentIn Ver. 2.1
<210> 1
   <211> 1030
   <212> DNA
   <213> künstliche Sequenz
<220>
   <223> Beschreibung der künstlichen Sequenz: scFv kodierende Sequenz
<400> 1
<210> 2
   <211> 927
   <212> DNA
   <213> künstliche Sequenz
<220>
   <223> Beschreibung der künstlichen Sequenz: scFv kodierende Sequenz
<210> 3
   <211> 990
   <212> DNA
   <213> künstliche Sequenz
<220>
   <223> Beschreibung der künstlichen Sequenz: scFv kodierende Sequenz
<400> 3
<210> 4
   <211> 946
   <212> DNA
   <213> künstliche Sequenz
<220>
   <223> Beschreibung der künstlichen Sequenz: scFv kodierende Sequenz
<400> 4
<210> 5
   <211> 906
   <212> DNA
   <213> künstliche Sequenz
<220>
   <223> Beschreibung der künstlichen Sequenz: scFv kodierende Sequenz
<400> 5
<210> 6
   <211> 329
   <212> PRT
   <213> Künstliche Sequenz
<220>
   <223> Beschreibung der künstlichen Sequenz: scFv von SEQ ID NO: 1 kodiert
<400> 6
   Met Asp Cys Leu Thr Asn Leu Arg Ser Ala Glu Gly Lys Val Asp Gln
   1               5                   10                  15
   Ala Ser Lys Ile Leu Ile Leu Leu Val Ala Trp Trp Gly Phe Gly Thr
               20                  25                  30
   Thr Ala Glu Val Ser Thr Ala Arg Ala Ala Gln Pro Ala Met Ala Glu
           35                  40                  45
   Val Lys Leu Gln Gln Ser Gly Ala Glu Leu Val Arg Pro Gly Val Ser
       50                  55                  60
   Val Lys Ile Ser Cys Lys Gly Ser Gly Tyr Thr Phe Thr Asp Tyr Gly
   65                  70                  75                  80
   Met Ser Trp Val Lys Gln Ser His Ala Lys Ser Leu Glu Trp Ile Gly
                   85                  90                  95
   Leu Ile Ser Thr Tyr Tyr Gly Asp Pro Ser Tyr Asn Gln Arg Phe Lys
               100                 105                 110
   Gly Lys Ala Thr Met Thr Val Asp Lys Ser Ser Asn Thr Ala Tyr Leu
           115                 120                 125
   Glu Leu Ala Arg Leu Thr Ser Glu Asp Ser Ala Ile Tyr Tyr Cys Ala
       130                 135                 140
   Arg Ser Asp Gly Asn Tyr Gly Tyr Tyr Tyr Ala Leu Asp Tyr Trp Gly
   145                 150                 155                 160
   Gln Gly Thr Thr Val Thr Val Ser Ser Gly Gly Gly Gly Ser Gly Gly
                   165                 170                 175
   Gly Gly Ser Gly Gly Gly Gly Ser Asp Ile Glu Leu Thr Gln Ser Pro
               180                 185                 190
   Ser Ser Leu Ala Val Ser Leu Gly Gln Arg Ala Thr Ile Ser Cys Arg
           195                 200                 205
   Ala Ser Glu Ser Val Asp Ser Tyr Gly Asp Ser Phe Met His Trp Tyr
       210                 215                 220
   Gln Gln Lys Pro Gly Gln Pro Pro Lys Leu Leu Ile Tyr Arg Ala Ser
   225                 230                 235                 240
   Asn Leu Glu Ser Gly Val Pro Ala Arg Phe Ser Gly Ser Gly Ser Glu
                   245                 250                 255
   Ser Asp Phe Thr Leu Thr Ile Asp Pro Val Glu Glu Asp Asp Ala Ala
               260                 265                 270
   Val Tyr Tyr Cys Leu Gln Ser Met Glu Asp Pro Tyr Thr Phe Gly Gly
           275                 280                 285
   Gly Thr Lys Leu Glu Ile Lys Arg Ala Ala Ala Ser Gly Ser Gly Gly
       290                 295                 300
   Gly Gly Ser Gly Gly Gly Ser Gly Gly Gly Gly Ser Gly Gly Gly Gly
   305                 310                 315                 320
   Ser Gly Ala Ser Pro Val Gln Phe Ile
                   325
<210> 7
<211> 309
   <212> PRT
   <213> Künstliche Sequenz
<220>
   <223> Beschreibung der künstlichen Sequenz: scFv von SEQ ID NO: 2 kodiert
<400> 7
   Met Asp Cys Leu Thr Asn Leu Arg Ser Ala Glu Gly Lys Val Asp Gln
   1               5                   10                  15
   Ala Ser Lys Ile Leu Ile Leu Leu Val Ala Trp Trp Gly Phe Gly Thr
               20                  25                  30
   Thr Ala Glu Val Ser Thr Ala Arg Ala Ala Gln Pro Ala Met Ala Glu
           35                  40                  45
   Val Lys Leu Gln Glu Ser Gly Thr Glu Leu Val Lys Pro Gly Ala Ser
       50                  55                  60
   Val Asn Leu Ser Cys Lys Ala Ser Gly Tyr Thr Phe Thr Ser Tyr Trp
   65                  70                  75                  80
   Met His Trp Leu Lys Gln Arg Pro Gly Gln Gly Leu Glu Trp Ile Gly
                   85                  90                  95
   Glu Ile Asp Pro Val Asp Ser Tyr Thr Asn Tyr Asn Gln Asn Phe Lys
               100                 105                 110
   Gly Lys Ala Thr Leu Thr Val Asp Lys Ser Ser Thr Thr Val Tyr Met
           115                 120                 125
   His Leu Ser Ser Leu Thr Ser Glu Asp Ser Ala Val Tyr Tyr Cys Ala
       130                 135                 140
   Arg Lys Gly Tyr Ala Met Asp Tyr Trp Gly Gln Gly Thr Asn Val Thr
   145                 150                 155                 160
   Val Ser Ser Gly Gly Cys Gly Ser Gly Gly Gly Gly Ser Gly Gly Gly
                   165                 170                 175
   Gly Ser Asp Ile Glu Leu Thr Gln Ser Pro Ala Ile Met Ser Ala Ser
               180                 185                 190
   Pro Gly Glu Lys Val Thr Met Thr Cys Ser Ala Ser Ser Ser Ile Ser
           195                 200                 205
   Tyr Met His Trp Tyr Gln Gln Lys Pro Gly Thr Ser Pro Lys Arg Trp
       210                 215                 220
   Ile Tyr Asp Thr Ser Lys Leu Ala Ser Gly Val Pro Ala Arg Phe Ser
   225                 230                 235                 240
   Gly Ser Gly Ser Gly Thr Ser Tyr Ser Leu Pro Ile Ser Ser Met Glu
                   245                 250                 255
   Ala Glu Asp Ala Ala Thr Tyr Tyr Cys His Gln Arg Ser Ser Tyr Pro
   260 265 270
   Trp Thr Phe Gly Gly Gly Thr Lys Leu Glu Ile Lys Arg Ala Ala Ala
   275 280 285
   Ser Gly Ser Gly Gly Gly Gly Ser Gly Gly Gly Ser Gly Gly Gly Gly
   290 295 300
   Ser Gly Gly Gly Gly
   305
<210> 8
   <211> 330
   <212> PRT
   <213> Künstliche Sequenz
<220>
   <223> Beschreibung der künstlichen Sequenz: scFv von SEQ ID NO: 3 kodiert
<400> 8
   Met Asp Cys Leu Thr Asn Leu Arg Ser Ala Glu Gly Lys Val Asp Gln
   1 5 10 15
   Ala Ser Lys Ile Leu Ile Leu Leu Val Ala Trp Trp Gly Phe Gly Thr
   20 25 30
   Thr Ala Glu Val Ser Thr Ala Arg Ala Ala Gln Pro Ala Met Ala Gln
   35 40 45
   Val Gln Leu Gln Gln Ser Gly Thr Glu Leu Ala Thr Pro Gly Ala Ser
   50 55 60
   Val Arg Met Ser Cys Lys Ala Ser Gly Tyr Ala Phe Thr Thr Tyr Trp
   65 70 75 80
   Met His Trp Val Lys Gln Arg Pro Gly Gln Gly Leu Glu Trp Ile Gly
   85 90 95
   Tyr Ile Asn Pro Thr Thr Asp Tyr Thr Asp Tyr Asn Leu Lys Phe Lys
   100 105 110
   Asp Lys Ala Thr Leu Thr Ala Asp Lys Ser Ser Ser Thr Ala Tyr Met
   115 120 125
   Gln Leu Ser Ser Leu Thr Ser Glu Asp Ser Ala Val Tyr Tyr Cys Ala
   130 135 140
   Arg Ser Gly Trp Ser Tyr Ala Met Asp Tyr Trp Gly Gln Gly Thr Thr
   145 150 155 160
   Val Thr Ile Ser Ser Gly Gly Gly Gly Ser Gly Gly Gly Gly Ser Gly
   165 170 175
   Gly Gly Gly Ser Asp Ile Glu Leu Thr Gln Ser Pro Ala Ile Met Ser
   180 185 190
   Ala Ser Pro Gly Glu Lys Val Thr Ile Thr Cys Ser Ala Ser Ser Ser
   195 200 205
   Val Ser Tyr Met His Trp Phe Gln Gln Lys Pro Gly Thr Ser Pro Lys
   210 215 220
   Leu Trp Ile Tyr Ser Thr Ser Asn Leu Ala Ser Gly Val Pro Ala Arg
   225 230 235 240
   Phe Ser Gly Ser Gly Ser Gly Thr Ser Tyr Ser Leu Thr Ile Ser Arg
   245 250 255
   Met Glu Ala Glu Asp Ala Ala Thr Tyr Tyr Cys Gln Gln Arg Ser Ser
   260 265 270
   Tyr Pro Phe Thr Phe Gly Ser Gly Thr Lys Leu Glu Ile Lys Arg Ala
   275 280 285
   Ala Ala Ser Gly Ser Gly Gly Gly Gly Ser Gly Gly Gly Gly Ser Gly
   290 295 300
   Gly Gly Gly Ser Gly Gly Gly Gly Ser Gly Ala Ser Pro Val Gln Phe
   305 310 315 320
   Ile Pro Leu Leu Val Gly Leu Gly Ile Ser
   325 330
<210> 9
   <211> 315
   <212> PRT
   <213> Künstliche Sequenz
<220>
   <223> Beschreibung der künstlichen Sequenz: scFV von SEQ ID NO: 4 kodiert
<400> 9
   Met Asp Cys Leu Thr Asn Leu Arg Ser Ala Glu Gly Lys Val Asp Gln
    1               5                  10                  15
   Ala Ser Lys Ile Leu Ile Leu Leu Val Ala Trp Trp Gly Phe Gly Thr
               20                  25                  30
   Thr Ala Glu Val Ser Thr Ala Arg Ala Ala Gln Pro Ala Met Ala Glu
           35                  40                  45
   Val Lys Leu Gln Gln Ser Gly Ala Glu Leu Val Arg Pro Gly Ala Ser
       50                  55                  60
   Val Lys Leu Ser Cys Lys Thr Ser Gly Phe Ser Phe Thr Ser Tyr Trp
   65                  70                  75                  80
   Met Asn Trp Val Lys Leu Arg Pro Gly Gln Gly Leu Glu Trp Ile Gly
                   85                  90                  95
   Met Ile His Pro Ser Asp Ser Glu Thr Ser Leu Thr Gln Arg Phe Lys
               100                 105                 110
   Asp Lys Ala Thr Leu Thr Val Asp Lys Ser Ser Ser Thr Ala Tyr Met
   115 120 125
   Gln Leu Ser Ser Pro Thr Ser Glu Asp Ser Ala Val Tyr Tyr Cys Ala
   130 135 140
   Arg Ser Leu Tyr Ala Asn Tyr Pro Ser Trp Phe Thr Tyr Trp Gly Gln
   145 150 155 160
   Gly Thr Thr Val Thr Val Ser Ser Gly Gly Gly Gly Ser Gly Gly Gly
   165 170 175
   Gly Ser Gly Gly Gly Gly Ser Asp Ile Glu Leu Thr Gln Ser Pro Thr
   180 185 190
   Thr Met Ala Ala Ser Pro Gly Glu Lys Ile Thr Ile Thr Cys Ser Ala
   195 200 205
   Ser Ser Ser Ile Ser Ser Asn Tyr Leu His Trp Tyr Gln Gln Lys Pro
   210 215 220
   Gly Phe Ser Pro Lys Leu Leu Ile Tyr Arg Thr Ser Asn Leu Ala Ser
   225 230 235 240
   Gly Val Pro Ala Arg Phe Ser Gly Ser Gly Ser Gly Thr Ser Tyr Ser
   245 250 255
   Leu Thr Ile Gly Thr Met Glu Ala Glu Asp Val Ala Thr Tyr Tyr Cys
   260 265 270
   Gln Gln Gly Ser Ser Ile Pro Tyr Thr Phe Gly Gly Gly Thr Lys Leu
   275 280 285
   Glu Ile Lys Arg Ala Ala Ala Ser Gly Ser Gly Gly Gly Gly Ser Gly
   290 295 300
   Gly Gly Gly Ser Gly Gly Gly Gly Ser Gly Gly
   305 310 315
<210> 10
   <211> 302
   <212> PRT
   <213> Künstliche Sequenz
<220>
   <223> Beschreibung der künstlichen Sequenz: scFV von SEQ ID NO: 5 kodiert
<400> 10
   Met Asp Cys Leu Thr Asn Leu Arg Ser Ala Glu Gly Lys Val Asp Gln
   1 5 10 15
   Ala Ser Lys Ile Leu Ile Leu Leu Val Ala Trp Trp Gly Phe Gly Thr
   20 25 30
   Thr Ala Glu Val Ser Thr Ala Arg Ala Ala Gln Pro Ala Met Ala Gln
           35                  40                  45
   Val Gln Leu Gln Gln Ser Gly Ala Glu Met Lys Lys Pro Gly Glu Ser
       50                  55                  60
   Leu Lys Ile Ser Cys Lys Gly Phe Gly Tyr Asp Phe Ser Thr Tyr Trp
   65                  70                  75                  80
   Ile Ala Trp Val Arg Gln Met Pro Gly Lys Gly Leu Glu Tyr Met Gly
                   85                  90                  95
   Leu Ile Tyr Pro Gly Asp Ser Asp Thr Lys Tyr Ser Pro Ser Phe Gln
               100                 105                 110
   Gly Gln Val Thr Ile Ser Ala Asp Lys Ser Ile Ser Thr Ala Tyr Leu
           115                 120                 125
   Gln Trp Ser Ser Leu Lys Ala Ser Asp Thr Ala Met Tyr Tyr Cys Ala
       130                 135                 140
   Arg Val Ser Gly Tyr Cys Ser Ser Thr Ser Cys Tyr Asp Tyr Tyr Tyr
   145                 150                 155                 160
   Tyr Tyr Met Asp Val Trp Gly Arg Gly Thr Leu Val Thr Val Ser Arg
                   165                 170                 175
   Gly Gly Gly Gly Ser Gly Gly Gly Gly Ser Gly Gly Gly Gly Ser Asp
               180                 185                 190
   Ile Val Met Thr Gln Ser Pro Ser Thr Leu Ser Ala Ser Val Gly Asp
           195                 200                 205
   Arg Val Thr Met Thr Cys Arg Ala Ser Gln Asn Ile Asn Ile Trp Leu
       210                 215                 220
   Ala Trp Tyr Gln Gln Lys Pro Gly Lys Ala Pro Lys Leu Leu Ile Tyr
   225                 230                 235                 240
   Lys Ala Ser Thr Leu Glu Ser Gly Val Pro Ser Arg Phe Ser Gly Ser
                   245                 250                 255
   Gly Ser Gly Thr Glu Phe Thr Leu Thr Ile Ser Gly Leu Gln Pro Asp
               260                 265                 270
   Asp Phe Ala Ser Tyr Tyr Cys Gln Arg Tyr Asp Ser Asp Trp Ser Phe
           275                 280                 285
   Gly Gln Gly Thr Lys Leu Glu Ile Lys Arg Ala Ala Ala Ser
       290                 295                 300